# EUROPEAN PATENT APPLICATION

(11) **EP 4 356 929 A1**
(43) Date of publication of application: **24.04.2024**
(21) Application number: 22202619.7
(22) Date of filing: 19.10.2022
(51) Int. Cl.: A61K 47/69, A61P 27/02, A61P 27/06, A61K 31/407, A61K 31/7048, A61K 9/00, A61K 9/06, A61K 47/40

(54) **ANTIFIBROTIC FORMULATION FOR OPHTHALMIC TREATMENT**

(71) Applicant: Universität Rostock, 18051 Rostock (DE)
(72) Inventor: Huling, Jennifer, 18055 Rostock (DE); Eickner, Thomas, 18059 Rostock (DE); Stefan Thomas, Oschatz, 18057 Rostock (DE); Grabow, Niels, 18055 Rostock (DE); Sterenczak, Katharina, 18055 Rostock (DE); Stahnke, Thomas, 26452 Sande (DE); Stachs, Oliver, 18057 Sildemow (DE); Füllen, Georg, 18057 Rostock (DE); Anselm, Jünemann, 91052 Erlangen (DE)
(74) Representative: Schulz Junghans Patentanwälte PartGmbB

(57) **Abstract**

The present invention relates to a formulation of josamycin or a related compound in a crosslinked poly- or oligosaccharide, and the use of this formulation in ophthalmic treatment of infection and fibrosis.

## Description

### Field

The present invention relates to a formulation of josamycin or a related compound in a crosslinked poly- or oligosaccharide, and the use of this formulation in ophthalmic treatment of infection and fibrosis.

### Background

Glaucoma is one of the most common causes of blindness and affects more than 3% of people over the age of 40 worldwide. The prevalence is only expected to increase in the future with rapidly increasing elderly populations. A major cause of glaucoma related blindness is increased intraocular pressure, which is the only treatable cause. It can be treated by the surgical creation of a drainage site in the eye, called a trabeculectomy. However, excessive fibrotic response after surgery can cause occlusion of these drainage pathways. Antifibrotic drugs are commonly used during and after the surgery to limit the fibrotic response.

The standard clinical therapy is the administration of cytotoxic drugs such as mitomycin C or 5-fluorouracil during or after surgery, usually topically via a sponge or with sub-conjunctival injections. These substances inhibit the proliferation of all types of cells, not just the fibroblasts, which can lead to undesirable side effects. Additionally, repetitive application is required to maintain therapeutic levels of drug, which can lead to inconsistent drug dosing and high rates of patient non-compliance.

Other strategies to address the limitations in the state of the art exist, but a γ-Cyclodextrin (γCD)-based degradable Josamycin (JM) depot for fibrosis prevention in opthalmologic applications has not been reported or claimed.

Previously published work demonstrated that the antibiotic drug Josamycin can also act as an antifibrotic agent. The drug's antifibrotic effect has been demonstrated on primary human Tenon fibroblasts and supports the idea that this drug could be used to provide both antibiotic and antibacterial protection after glaucoma surgery, potentially with fewer side effects.

However, JM is poorly water soluble, making it difficult to administer locally in physiologically effective doses. Therefore, some type of biocompatible carrier is needed to facilitate the delivery of sufficiently high quantities of JM to the eye after glaucoma surgery.

Degradable solid drug eluting implants for use in the eye are known to a person skilled in the art. Example products include OZURDEX^{®}, DEXYCU^{®} and DEXTENZA^{®}, which are all used to deliver the anti-inflammatory drug, dexamethasone. However, these products are not geared toward antifibrotic or glaucoma treatment. There is also research for use as solid drug delivery devices (Haley et al. 2020; Machín et al. 2013). Also, there exists research into utilizing yCD as a delivery vehicle for topical drug delivery to the eye in the form of eye drops (Loftsson und Stefánsson 2007; Loftsson und Stefánsson 2017). WO2016193810A1 discloses methods of forming cyclosporin/cyclodextrin complex nanoparticles and microparticles, and administration of the nano- and microsuspension formed to an eye of a human or animal in the form of aqueous eye drops. Document US8128954B2 discloses sustained-release biodegradable polymeric drug-eluting fiber. US8999953B2 discloses an ophthalmic composition, which is an aqueous suspension comprising drug, cyclodextrin and water. However, there are no commercial products composed of CD-based hydrogels.

Based on the above-mentioned state of the art, the objective of the present invention is to provide means and methods for an ophthalmic formulation for treatment of fibrosis. This objective is attained by the subject-matter of the independent claims of the present specification, with further advantageous embodiments described in the dependent claims, examples, figures and general description of this specification.

### Summary of the Invention

A first aspect of the invention relates to a composition comprising:
a. a crosslinked saccharide, wherein the saccharide consists of a polysaccharide and/or an oligosaccharide, the saccharide comprises cyclodextrin, and the saccharide is crosslinked via a crosslinking agent; and
b. an active pharmaceutical drug compound selected from the group consisting of josamycin, kitasamycin, and mitomycin C.

A further aspect of the invention relates to a composition according to the first aspect for use as a medicament.

A further aspect of the invention relates to a composition according to the first aspect for use in treatment or prevention of fibrosis of the eye and/or the conjunctiva.

A further aspect of the invention relates to a composition according to the first aspect for use in treatment or prevention of infection of the eye and/or the conjunctiva.

### Terms and definitions

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of' or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *crosslinking agent* in the context of the present specification relates to an agent being capable of forming a cross-link. A cross-link is a bond or a short sequence of bonds that links one molecule to another. The crosslinking agent covalently links the poly- or oligosaccharide subunits.

The term *josamycin* in the context of the present specification relates to a compound of CAS-Number 16846-24-5. The efficacy of josamycin in prevention or treatment of fibrosis is disclosed in WO2021186041 which is incorporated by reference herein.

The term *kitasamycin* in the context of the present specification relates to a compound of CAS-Number 22875-15-6. The efficacy of kitasamycin in prevention or treatment of fibrosis is disclosed in WO2021186041 which is incorporated by reference herein.

The term *mitomycin* C in the context of the present specification relates to a compound of CAS-Number 50-07-7.

The term *cyclodextrin* in the context of the present specification relates to a family of cyclic oligosaccharides, consisting of a macrocyclic ring of glucose subunits joined by α-1,4 glycosidic bonds. One ring comprises 6 glucose subunits in the case of α-cyclodextrin, 7 glucose subunits in the case of β-cyclodextrin, and 8 glucose subunits in the case of γ-cyclodextrin.

The term *dry crosslinked saccharide* in the context of the present specification relates to the weight of the saccharide only, not including the weight of the water associated with the saccharide.

The term *hydrogel* in the context of the present specification relates to a crosslinked hydrophilic polymer that does not dissolve in water. Hydrogels have two main regimes of mechanical properties: rubber elasticity and viscoelasticity. Rubber elasticity refers to a property of crosslinked rubber: it can be stretched by up to a factor of 10 from its original length and, when released, returns very nearly to its original length. Viscoelasticity is the property of materials that exhibit both viscous and elastic characteristics when undergoing deformation.

The term *molar ratio* in the context of the present specification relates to the quotient between the number of molecules of the firstly mentioned entity and the number of molecules of the secondly mentioned entity.

The term *ocular injection* in the context of the present specification relates to the method of administration of drugs into the eye by injection with a fine needle.

Any patent document cited herein shall be deemed incorporated by reference herein in its entirety.

As used herein, the term *pharmaceutical composition* refers to a compound of the invention, or a pharmaceutically acceptable salt thereof, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

As used herein, the term *treating* or *treatment* of any disease or disorder (e.g. fibrosis) refers in one embodiment, to ameliorating the disease or disorder (e.g. slowing or arresting or reducing the development of the disease or at least one of the clinical symptoms thereof). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

### Detailed Description of the Invention

A first aspect of the invention relates to a composition comprising:
a. a crosslinked saccharide, wherein the saccharide consists of a polysaccharide and/or an oligosaccharide, the saccharide comprises cyclodextrin, and the saccharide is crosslinked via a crosslinking agent; and
b. an active pharmaceutical drug compound selected from the group consisting of josamycin, kitasamycin, and mitomycin C.

In certain embodiments, the cyclodextrin is selected from the group consisting of α-cyclodextrin, β-cyclodextrin, and γ-cyclodextrin. In certain embodiments, the cyclodextrin is γ-cyclodextrin.

In certain embodiments, the crosslinked saccharide forms a hydrogel. The hydrogel permits release of the compound in the course of several days.

In certain embodiments, the crosslinked saccharide additionally comprises another saccharide than cyclodextrin. In certain embodiments, the crosslinked saccharide additionally comprises a saccharide selected from the group consisting of dextrin, dextran, starch, chitosan, chitin, cellulose, pectin, alginate, hyaluronic acid, agar, pullulan, and gellan gum. In certain embodiments, the crosslinked saccharide additionally comprises dextran.

In certain embodiments, the active pharmaceutical drug compound is josamycin.

In certain embodiments, the crosslinking agent is selected from the group consisting of epichlorohydrin, bis[2-(succinimidyloxycarbonyloxy)ethyl]sulfone (BSOCOES), disuccinimidyl suberate (DSS), di-(N-succinimidyl)-carbonate (DCS), ethylenediaminetetraacetic acid (EDTA), sodium trimetaphosphate, divinyl sulfone, glutaraldehyde, 1-ethyl-3-(-3-dimethylaminopropyl) carbodiimide hydrochloride, hexamethylene diisocyanate (HDI) and N', N'-dicyclohexyl carbodiimide. In certain embodiments, the crosslinking agent is epichlorohydrin.

In certain embodiments, the molar ratio before crosslinking of the saccharide:crosslinking agent is in a range of 1:1 to 1:50. In certain embodiments, the molar ratio before crosslinking of the saccharide:crosslinking agent is in a range of 1:2 to 1:35. In certain embodiments, the molar ratio before crosslinking of the saccharide:crosslinking agent is in a range of 1:5 to 1:20.

In certain embodiments, the composition comprises 1-500 µg of the drug compound per 1000 µg of dry crosslinked saccharide. In certain embodiments, the composition comprises 5-100 µg of the drug compound per 1000 µg of dry crosslinked saccharide. In certain embodiments, the composition comprises -20-40 µg of the drug compound per 1000 µg of dry crosslinked saccharide.

A further aspect of the invention relates to a composition according to the first aspect for use as a medicament.

A further aspect of the invention relates to a composition according to the first aspect for use in treatment or prevention of fibrosis of the eye and/or the conjunctiva.

In certain embodiments, the fibrosis is caused by, or occurs subsequent to, glaucoma surgery.

A further aspect of the invention relates to a composition according to the first aspect for use in treatment or prevention of infection of the eye and/or the conjunctiva.

In certain embodiments, the composition is administered during or after glaucoma surgery.

In certain embodiments, the composition is administered via ocular injection.

In certain embodiments, the composition is administered as an implant or as a drug delivery system to ensure local and sustained drug release over several days. In certain embodiments, the composition is administered via placement on the site of surgery by the surgeon. In certain embodiments, the implant is placed into the subconjunctival space. The subconjunctival space is the hydrophilic, fluid-filled space between the conjunctiva and the sclera.

A drug delivery system is a combination of a carrier or vehicle with a therapeutic agent, which enables the controlled release of the therapeutic agent to suit the temporal or spatial requirements of the specific treatment.

### Medical treatment

Similarly, within the scope of the present invention is a method or treating fibrosis or infection of the eye and/or the conjunctiva in a patient in need thereof, comprising administering to the patient a formulation comprising a crosslinked saccharide and a compound selected from the group comprising josamycin, kitasamycin, and mitomycin C according to the above description.

### Pharmaceutical Compositions, Administration/Dosage Forms and Salts

According to one aspect of the compound according to the invention, the compound according to the invention is provided as a pharmaceutical composition, pharmaceutical administration form, or pharmaceutical dosage form, said pharmaceutical composition, pharmaceutical administration form, or pharmaceutical dosage form comprising at least one of the compounds of the present invention or a pharmaceutically acceptable salt thereof and at least one pharmaceutically acceptable carrier, diluent or excipient.

The skilled person is aware that any specifically mentioned drug compound mentioned herein may be present as a pharmaceutically acceptable salt of said drug. Pharmaceutically acceptable salts comprise the ionized drug and an oppositely charged counterion. Non-limiting examples of pharmaceutically acceptable anionic salt forms include acetate, benzoate, besylate, bitatrate, bromide, carbonate, chloride, citrate, edetate, edisylate, embonate, estolate, fumarate, gluceptate, gluconate, hydrobromide, hydrochloride, iodide, lactate, lactobionate, malate, maleate, mandelate, mesylate, methyl bromide, methyl sulfate, mucate, napsylate, nitrate, pamoate, phosphate, diphosphate, salicylate, disalicylate, stearate, succinate, sulfate, tartrate, tosylate, triethiodide and valerate. Non-limiting examples of pharmaceutically acceptable cationic salt forms include aluminium, benzathine, calcium, ethylene diamine, lysine, magnesium, meglumine, potassium, procaine, sodium, tromethamine and zinc.

In certain embodiments of the invention, the compound of the present invention is typically formulated into pharmaceutical dosage forms to provide an easily controllable dosage of the drug and to give the patient and/or the surgeon an elegant and easily handleable product.

Similarly, a dosage form for the prevention or treatment of fibrosis or infection of the eye and/or the conjunctiva is provided, comprising a formulation according to any of the above aspects or embodiments of the invention.

The invention further encompasses a pharmaceutical composition comprising a compound of the present invention, or a pharmaceutically acceptable salt thereof, and a pharmaceutically acceptable carrier. In further embodiments, the composition comprises at least two pharmaceutically acceptable carriers, such as those described herein.

The dosage regimen for the drug compounds of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the drug compounds of the invention may be administered in a single dose, a single monthly dose, a single weekly dose, a single daily dose, or the total daily dosage may be administered in divided doses of two, three, or four times daily.

The therapeutically effective dosage of a drug compound, the pharmaceutical composition, or the combinations thereof, is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The pharmaceutical compositions of the present invention can be subjected to conventional pharmaceutical operations such as sterilization and/or can contain conventional inert diluents, lubricating agents, or buffering agents, as well as adjuvants, such as preservatives, stabilizers, wetting agents, emulsifiers and buffers, etc. They may be produced by standard processes, for instance by conventional mixing, granulating, dissolving or lyophilizing processes. Many such procedures and methods for preparing pharmaceutical compositions are known in the art, see for example L. Lachman et al. The Theory and Practice of Industrial Pharmacy, 4th Ed, 2013 (ISBN 8123922892).

### Method of Manufacture and Method of Treatment according to the invention

The invention further encompasses, as an additional aspect, the use of a formulation comprising a crosslinked saccharide and an active pharmaceutical drug compound selected from the group comprising josamycin, kitasamycin, and mitomycin C, or its pharmaceutically acceptable salt, as specified in detail above, for use in a method of manufacture of a medicament for the treatment or prevention of fibrosis or infection of the eye and/or the conjunctiva.

Similarly, the invention encompasses methods of treatment of a patient having been diagnosed with a disease associated with fibrosis or infection of the eye and/or the conjunctiva. This method entails administering to the patient an effective amount of a formulation comprising a crosslinked saccharide and an active pharmaceutical drug compound selected from the group comprising josamycin, kitasamycin, and mitomycin C as identified herein, or its pharmaceutically acceptable salt, as specified in detail herein.

Wherever alternatives for single separable features such as, for example, an active pharmaceutical drug compound, or a type of poly- or oligosaccharide or a medical indication are laid out herein as "embodiments", it is to be understood that such alternatives may be combined freely to form discrete embodiments of the invention disclosed herein. Thus, any of the alternative embodiments for a drug compound may be combined with any of the alternative embodiments of a type of poly- or oligosaccharide and these combinations may be combined with any medical indication mentioned herein.

The invention is further illustrated by the following examples and figures, from which further embodiments and advantages can be drawn. These examples are meant to illustrate the invention but not to limit its scope.

### Description of the Figures

- Fig. 1: shows the process of creating the solid formulation. γCD molecules were crosslinked with epichlorohydrin (EPI) to form a hydrogel. This hydrogel can then be loaded with JM, which forms a host-guest complex with the yCD molecules
- Fig. 2: the amount of chlorine in various EPI crosslinked hydrogel samples was quantified. The EPI contains chlorine, but the final crosslinked hydrogel should not. Gels washed at 37°C in DI water were given either three 1-hour washes (short wash) or two 1-hour washes followed by an overnight wash with water or Ethanol. In all cases, the amount of residual chlorine was significantly lower than in the unwashed gel samples.
- Fig. 3: the maximum solubility of Josamycin in aqueous solutions of various yCD concentrations was measured. Soluble JM was measured by HPLC.
- Fig. 4: shows that JM loaded yCD hydrogel was incubated in PBS at 37°C. The amount of JM released was measured over time and expressed as a percent of the total JM initially loaded into the gel. Most of the JM was released within the first 96 hrs.
- Fig. 5: shows eluent cytotoxicity experiments. The left graph shows cell viability measured by the RealTime-Glo assay in arbitrary luminescence units. The right graph shows cell toxicity measured by the CellTox Green in arbitrary fluorescent units.
- Fig. 6: shows macroscopic images of examples of the γ-CD hydrogels. On the left are molded and washed gels, which have been completely dried. On the right is a fully rehydrated gel which is almost completely transparent.
- Fig. 7: shows the average swelling percent of γCD hydrogels made with a γCD:EPI 1:10 ratio as described previously (n=30 gels).
- Fig. 8: shows the average total loaded josamycin in -12 mm x 1 mm cylindrical hydrated yCD gels after soaking overnight in loading solution (n=30).
- Fig. 9: shows that yCD gels were drug loaded overnight with solutions of 0.9, 0.44 or 0.21 mg/ml Josamycin and the total loaded drug was measured (n=3 per concentration). The total drug loading was linearly related to the amount of josamycin in the loading solution.

### Examples

### Example 1: Manufacturing steps

An implant is described as follows:
1. Consisting of a drug loaded hydrogel from crosslinked oligosaccharide, in particular gamma-cyclodextrin, or a solution of gamma-cyclodextrin
2. where the drug is from the macrolide antibiotic substance class, in particular Josamycin with an off-label use as an antifibrotic drug,
3. where the crosslinker is epichlorohydrin (tentative molar ratio of γCD:EPI range: 1:5-1:20)
4. and the drug is entrapped into the crosslinked cyclic oligosaccharide or the dissolved oligosaccharide
5. showing a sustained drug release for at least 100 hours
6. for ophthalmologic usage, in particular as an implant during trabeculectomy surgeries to be placed into the subconjunctival space
7. where the cyclic oligosaccharide is biodegradable and be fully absorbed by the body after complete drug release

The first formulation developed is a solution of yCD and JM. Solutions of JM and yCD can be made up to 18.9 mg/ml JM with 200 mg/ml yCD. These solutions are formed by simply mixing the JM and yCD in water or PBS until dissolved. This liquid formulation can then be sterilized via syringe filter before use. The solution is suitable for application to the eye as an eye drop or injection during or after surgery.

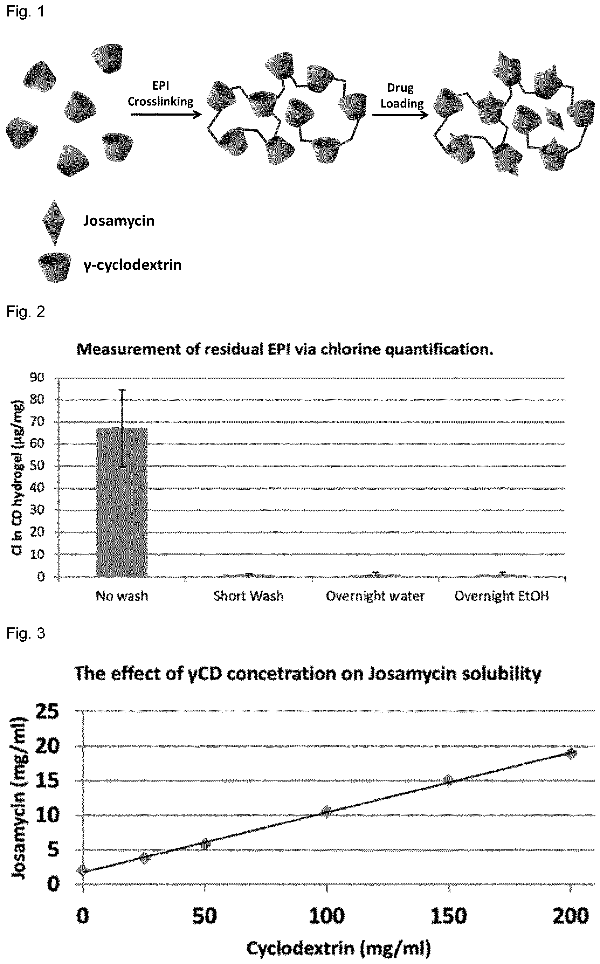

The second formulation developed is in the form of a solid implantable hydrogel. The formulation still capitalizes on the interaction of yCD and JM, but crosslinks the yCD molecules together to form a gel. γCD can be crosslinked directly with epichlorohydrin (EPI). The crosslinking process first forms long polymer chains of cyclodextrins and then crosslinking between chains forms a hydrogel (figure 1). The protocol developed for this formulation was based on parameters which demonstrated gel formation in other published works (Wintgens und Amiel 2010).

To create the hydrogel, yCD was dissolved in 30% w/v NaOH to form a final 50% w/v solution. EPI was added to a final molar ratio of γCD:EPI of 1:10. The mixture was stirred at room temperature until homogeneous and then cured overnight at room temperature. The mixture could be dispensed into glass or silicone molds to create hydrogels of a controlled size and shape.

The final hydrogels were rinsed in deionized water at 37°C. The washing procedure consists of two 1-hour rinses followed by an overnight rinse which was found to be sufficient to remove the unreacted EPI. Several rinsing procedures were tested and all were found to remove EPI based on residual chorine amounts in the washed gels (Figure 2).

After washing, the hydrogels were dried. JM was loaded by immersing the dried hydrogels in a 1 mg/ml solution of JM for 24 at room temperature. Then the drug loaded samples were dried again for storage until use.

γCD is a biocompatible and biodegradable cyclic oligosaccharide. Its ring-like structure has a hydrophilic outer surface, making it highly water soluble, and a hydrophilic inner surface, which can form inclusion complexes with hydrophobic drugs. γCD can form an inclusion complex with JM and significantly increase the concentration of the poorly water-soluble JM in aqueous solutions. The effect of yCD on JM concentration is shown in Figure 3.

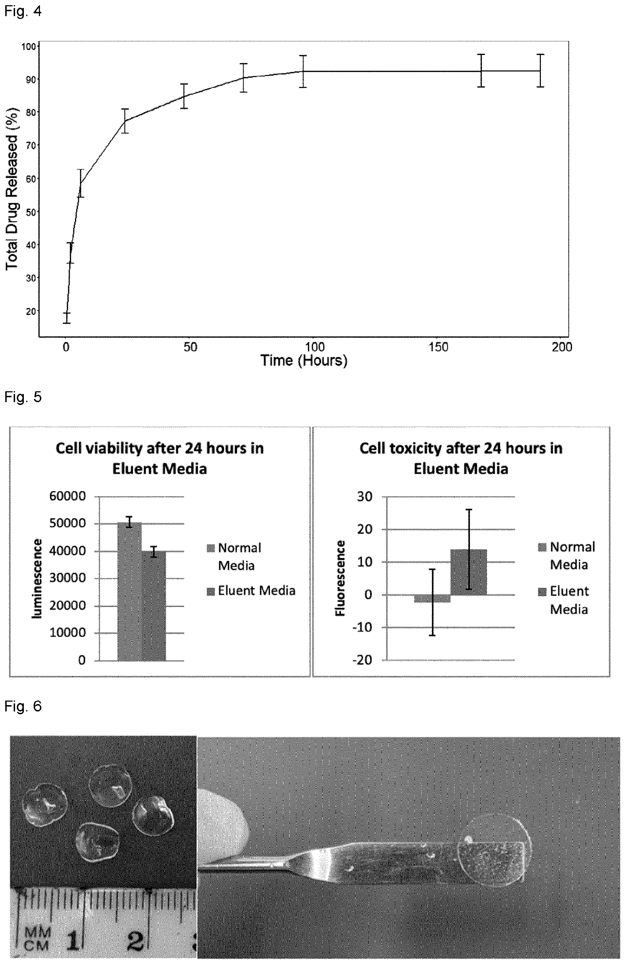

yCD was crosslinked with EPI to form hydrogels. -12 mm x 1 mm disks of hydrogel were loaded with drug and then placed in PBS at 37°C to generate a release curve. The release of JM from the hydrogel can be seen in Figure 4. The drug was released over the first 96 hrs and more than 90% of the total loaded drug was released over the course of 8 days.

An initial evaluation of biocompatibility was performed using a mouse fibroblasts cell line (L929). After 24 hours under normal culture conditions, eluent media were added and the cells were cultured for an additional 24 hours. Eluent media were prepared by soaking washed yCD hydrogel samples in the normal growth media for 48 hours at 37°C before use. Cell viability and toxicity were measured using the multiplexed assays of Realtime-Glo and CellTox Green assays (Figure 5). The cells grown in eluent media for 24hrs showed 78.6% viability compared to normal cells and negligible toxicity. This initial finding supports the biocompatibility of yCD hydrogels and shows that after washing they are no longer leaching any unreacted toxic products.

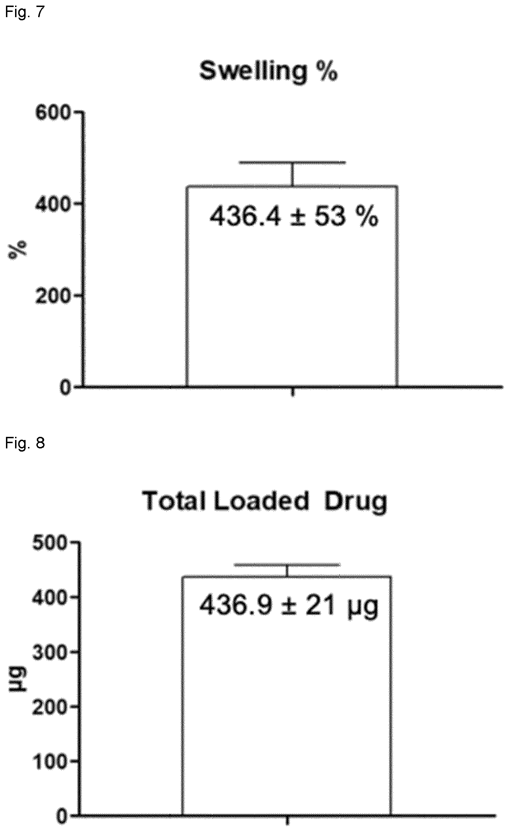

The swelling percent of γCD hydrogels made as described previously (γCD:EPI 1:10), was measured. Water uptake is a defining characteristic of hydrogels and critical to drug loading and release properties. The γCD gels showed an average swelling of 436.4 ± 53 %. Hydrogels can span a very wide range of swelling percentages which the inventors would be able to influence by adjusting the chosen materials, crosslinking method and crosslinking amount. It would be reasonable to expect final gel swelling percentages between 5-5000% (Fig. 7).

The yCD gels were loaded with josamycin by soaking them overnight in a saturated solution of josamycin in water. The amount of drug loaded into the γCD gels was measured and resulted in an average of 436.9 ± 21 µg josamycin loaded into an approximately 12 mm x 1 mm cylindrical hydrated γCD gel. Normalized to the mass of the gel, this is 19.87 µg of josamycin loaded per 1000 µg of dried γCD gel (Fig. 8).

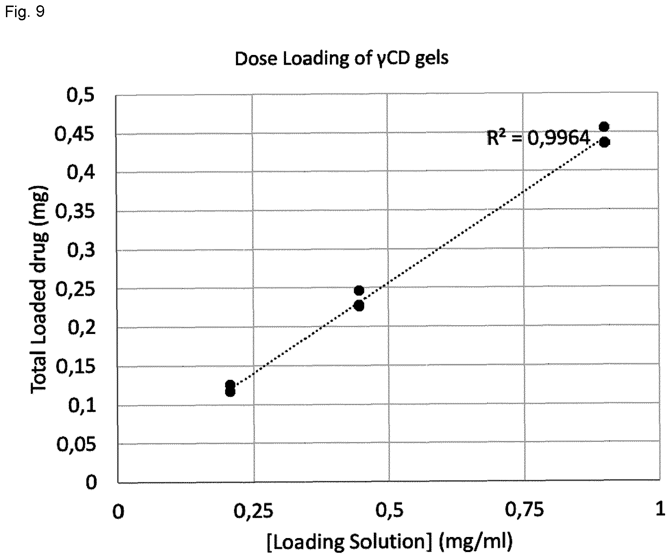

The inventors have also shown that the amount of josamycin loaded into γCD gels is linearly proportional to the concentration of the josamycin in the loading solution. Therefore, the total amount of loaded drug can be easily adjusted based on this linear relationship by adjusting the drug loading solution concentration and the total loaded amount could be theoretically increased beyond what was measured here by increasing the loading solution concentration (Fig. 9).

The novelty of this invention is in creating a topical or injectable solution and implantable drug delivery system specifically for Josamycin after glaucoma surgery to prevent fibrosis. Local delivery and extended release from a drug depot would eliminate the need for multiple drug applications after surgery through injection or eye drops. Utilizing γCD as a carrier molecule targets the current limitation of JM, which is its low solubility. Using γCD in solution with JM or the form of an implantable hydrogel should improve drug loading efficacy because γCD forms an inclusion complex with JM. Additionally, JM may prove to be less cytotoxic than current standard treatment, while providing both antibiotic and antifibrotic effects. The inventors believe that this technique could be used to create a more patient friendly off-the-shelf product that provides improved clinical efficacy. To the best of the author's knowledge, there are no existing products or research papers, which use JM loaded γCD hydrogels for the treatment of fibrosis after trabeculectomy.

### Cited prior art documents:

All scientific publications and patent documents cited in the present specification are incorporated by reference herein.

Haley, Rebecca M.; Zuckerman, Sean T.; Dakhlallah, Hassan; Capadona, Jeffery R.; Recum, Horst A. von; Ereifej, Evon S. (2020): Resveratrol Delivery from Implanted Cyclodextrin Polymers Provides Sustained Antioxidant Effect on Implanted Neural Probes. In: International journal of molecular sciences 21 (10). DOI: 10.3390/ijms21103579.

Loftsson, T.; Stefánsson, E. (2007): Cyclodextrins in ocular drug delivery: theoretical basis with dexamethasone as a sample drug. In: Journal of Drug Delivery Science and Technology 17 (1), S. 3-9. DOI: 10.1016/S1773-2247(07)50001-8.

Loftsson, Thorsteinn; Stefánsson, Einar (2017): Cyclodextrins and topical drug delivery to the anterior and posterior segments of the eye. In: International Journal of Pharmaceutics 531 (2), S. 413-423. DOI: 10.1016/j.ijpharm.2017.04.010.

Machín, Rubén; Isasi, Jose Ramon; Vélaz, Itziar (2013): Hydrogel matrices containing single and mixed natural cyclodextrins. Mechanisms of drug release. In: European Polymer Journal 49 (12), S. 3912-3920. DOI: 10.1016/j.eurpolymj.2013.08.020.

Wintgens, Véronique; Amiel, Catherine (2010): Water-soluble γ-cyclodextrin polymers with high molecular weight and their complex forming properties. In: European Polymer Journal 46 (9), S. 1915-1922. DOI: 10.1016/j.eurpolymj.2010.06.014.

## Claims

1. A composition comprising:
a. a crosslinked saccharide, wherein
i. the saccharide consists of a polysaccharide and/or an oligosaccharide,
ii. the saccharide comprises cyclodextrin, and
iii. the saccharide is crosslinked via a crosslinking agent;
and
b. a drug compound selected from the group consisting of josamycin, kitasamycin, and mitomycin C.

2. The composition according to claim 1, wherein said cyclodextrin is selected from the group consisting of α-cyclodextrin, β-cyclodextrin, and γ-cyclodextrin, more particularly wherein said cyclodextrin is γ-cyclodextrin.

3. The composition according to any one of the preceding claims, wherein said crosslinked saccharide forms a hydrogel.

4. The composition according to any one of the preceding claims, wherein said crosslinked saccharide additionally comprises another saccharide in addition to cyclodextrin, particularly wherein said crosslinked saccharide additionally comprises a saccharide selected from the group consisting of dextrin, dextran, starch, chitosan, chitin, cellulose, pectin, alginate, hyaluronic acid, agar, pullulan, and gellan gum.

5. The composition according to any one of the preceding claims, wherein said drug compound is josamycin.

6. The composition according to any one of the preceding claims, wherein said crosslinking agent is selected from the group consisting of epichlorohydrin, bis[2-(succinimidyloxycarbonyloxy)ethyl]sulfone (BSOCOES), disuccinimidyl suberate (DSS), di-(N-succinimidyl)-carbonate (DCS), ethylenediaminetetraacetic acid (EDTA), sodium trimetaphosphate, divinyl sulfone, glutaraldehyde, 1-ethyl-3-(-3-dimethylaminopropyl) carbodiimide hydrochloride, hexamethylene diisocyanate (HDI) and N', N'-dicyclohexyl carbodiimide, particularly wherein said crosslinking agent is epichlorohydrin.

7. The composition according to according to any one of the preceding claims, wherein the molar ratio before crosslinking of said saccharide:crosslinking agent is in a range of 1:1 to 1:50, particularly in a range of 1:2 to 1:35, particularly in a range of 1:5 to 1:20.

8. The composition according to any one of the preceding claims, wherein the composition comprises 1-500 µg of the drug compound per 1000 µg of dry crosslinked saccharide, particularly wherein the composition comprises 5-100 µg of the drug compound per 1000 µg of dry crosslinked saccharide, more particularly wherein the composition comprises -20-40 µg of the drug compound per 1000 µg of dry crosslinked saccharide.

9. A composition according to any one of the preceding claims 1 to 8 for use as a medicament.

10. A composition according to any one of the preceding claims 1 to 8 for use in treatment or prevention of fibrosis of the eye and/or the conjunctiva.

11. The composition for use according to claim 10, wherein the fibrosis is caused by, or occurs subsequent to, glaucoma surgery.

12. A composition according to any one of the preceding claims 1 to 8 for use in treatment or prevention of infection of the eye and/or the conjunctiva.

13. The composition for use according to claims 9 to 12, wherein the composition is administered during or after glaucoma surgery.

14. The composition for use according to any one of claims 9 to 13, wherein the composition is administered via ocular injection.

15. The composition for use according to any one of claims 9 to 13, wherein the composition is administered as an implant or as a drug delivery system, particularly wherein the implant is placed into the subconjunctival space.
